# EUROPÄISCHE PATENTSCHRIFT

(11) **EP 3 410 879 B1**
(45) Veröffentlichungstag und Bekanntmachung des Hinweises auf die Patenterteilung: **21.10.2020**
(21) Anmeldenummer: 17723384.8
(22) Anmeldetag: 15.05.2017
(51) Int. Cl.: A41D 13/005, A41B 11/00

(54) **FUSSBEKLEIDUNG, INSBESONDERE SOCKEN**
FOOTWEAR, IN PARTICULAR SOCKS
ARTICLE CHAUSSANT, EN PARTICULIER CHAUSSETTE

(30) Priorität: 19.05.2016 AT 2532016
(43) Veröffentlichungstag der Anmeldung: 12.12.2018
(73) Patentinhaber: LENZ GES.M.B.H., 6858 Schwarzach (Vbg.) (AT)
(72) Erfinder: SCHABERREITER, Martin, 8253 Waldbach (AT); LENZ, Stefan, 6858 Schwarzach (Vbg.) (AT); KREMER, Gerhard, 8280 Fürstenfeld (AT)
(74) Vertreter: Beer & Partner Patentanwälte KG
(86) Internationale Anmeldenummer: PCT/EP2017/061555
(87) Internationale Veröffentlichungsnummer: WO 2017/198583

(56) Entgegenhaltungen:
- EP-A1- 2 481 309
- KR-U- 20110 001 806
- NL-C- 1 040 557

## Beschreibung

Die Erfindung betrifft eine Fußbekleidung, insbesondere Socken, umfassend einen Schaft mit einem Schaftende zur Aufnahme eines Unterbeines, einen am Schaft angeordneten Fußteil mit einer Oberseite und einer Unterseite zur Aufnahme eines Fußes, und einen am Fußteil angeordneten Umlenkbereich zur Aufnahme von Zehen, wobei die Fußbekleidung wenigstens ein Heizelement mit Anschlüssen für eine Spannungsquelle aufweist und wobei das Heizelement im Bereich des Fußteiles an der Oberseite des Fußteiles angeordnet : ist und eine Heizzone aufweist, dadurch gekennzeichnet, dass die Heizzone des Heizelements innen am Umlenkbereich angeordnet ist, so dass Zehen eines in der Fußbekleidung aufgenommenen Fußes vom Heizelement umschlossen werden.

Zudem betrifft die Erfindung ein Verfahren zur Herstellung einer Fußbekleidung.

Eine gattungsgemäße Fußbekleidung, insbesondere ein Socken oder Strumpf, ist aus KR 2011 0001806 U und NL 1 040 557 C bekannt. Bei bisher bekannten Socken oder Strümpfen mit integriertem Heizelement verläuft das Heizelement in Gebrauchslage unterhalb des Fußes. Um das Heizelement in dieser Lage im Socken anbringen zu können, ist der Socken, wenn er von der Strickmaschine kommt, oberhalb der Zehen offen. Das Heizelement wird dann im offenen Zustand des Sockens eingenäht und die oberhalb der Zehen liegende Öffnung wird durch eine Naht verschlossen. Ein solches Heizelement weist ein Heizzone auf, in der ein Heizdraht meist spiralförmig angeordnet ist. Besonders nachteilig ist dabei, dass man bei Verwendung eines solchen Sockens auf dem Heizelement steht. Dadurch wird der Tragekomfort vermindert und die Gefahr von Blasenbildung in entsprechenden Bereichen der Fußsohle oder der Ferse aufgrund von Druck und Reibung erhöht. Zudem steigt das Risiko, dass das Heizelement, z.B. durch Brechen eines elektrischen Leiters, beschädigt wird, was zu einem gänzlichen Funktionsverlust des Heizelementes sowie zu Reklamationen führt.

Der Erfindung liegt die Aufgabe zu Grunde, eine Fußbekleidung mit integriertem Heizelement sowie ein Verfahren zur Herstellung einer solchen Fußbekleidung bereitzustellen, mit welchen Nachteile des Standes der Technik vermieden werden.

Gelöst wird diese Aufgabe erfindungsgemäß mit einer Fußbekleidung, welche die Merkmale von Anspruch 1 aufweist, und mit einem Verfahren zur Herstellung einer solchen Fußbekleidung, welches die Merkmale von Anspruch 11 aufweist.

Bevorzugte und vorteilhafte Ausführungsformen der Erfindung sind Gegenstand der Unteransprüche.

Erfindungsgemäß ist vorgesehen, dass die Heizzone des Heizelements innen am Umlenkbereich angeordnet ist, so dass Zehen eines in der Fußbekleidung aufgenommenen Fußes vom Heizelement umschlossen werden. Wenn das Heizelement von der Oberseite des Fußteiles in den Umlenkbereich zur Aufnahme der Zehen verläuft und die wenigstens einen Heizdraht aufweisende Heizzone des Heizelementes, im Umlenkbereich angeordnet ist, so dass Zehen eines in der Fußbekleidung steckenden Fußes zumindest teilweise oder sogar zur Gänze von dem Heizelement bzw. der Heizzone umschlossen werden, kann der Tragekomfort erhöht und das Risiko einer Beschädigung und eines daraus resultierenden Funktionsverlustes des Heizelements vermindert werden, wodurch sich wiederum die Anzahl an zu erwartenden Reklamationen verringert.

In einer bevorzugten Ausführungsform ist vorgesehen, dass das Heizelement wenigstens eine Trägerlage aufweist, die vorzugsweise elastisch ausgeführt und in eine Richtung längenveränderbar bzw. dehnbar ist, und dass an oder in der Trägerlage wenigstens ein elektrischer Leiter angeordnet ist. Der elektrische Leiter ist vorzugsweise zumindest bereichsweise, wellenförmig an oder in der Trägerlage angeordnet. Durch die wellenförmige Anordnung des Leiters kann der an sich unelastische, elektrische Leiter zusammen mit der Trägerlage einer Längenveränderung (gedehnt oder gestaucht) unterzogen werden, ohne dadurch Schaden zu nehmen.

Insbesondere ist im Rahmen der Erfindung eine Ausführungsform bevorzugt, bei welcher der elektrische Leiter des Heizelementes mit der Trägerlage verbunden ist. Der Leiter kann dabei auf der Trägerlage aufgestickt und/oder aufgeklebt sein. Besonders bevorzugt ist es, wenn der Leiter an einer Oberfläche und/oder in die Trägerlage eingewebt und/oder eingewirkt ist, so dass ein in der Fußbekleidung steckender Fuß nur mit der Trägerlage und nicht mit Sticknähten oder Klebstellen in Kontakt kommt.
In einer vorteilhaften Ausführungsform ist vorgesehen, dass das Heizelement aus einer Zuleitung und einer Heizzone besteht, wobei der elektrische Leiter des Heizelementes im Bereich der Zuleitung einen vorzugsweise niedrigeren ohmschen Widerstand aufweist als im Bereich der Heizzone. Dadurch wirkt der elektrische Leiter bei Anschluss einer elektrischen Spannungsquelle an den elektrischen Leiter im Bereich der Heizzone als Heizdraht und wandelt die angelegte elektrische Energie zumindest teilweise in thermische Energie um.

Insbesondere ist im Rahmen der Erfindung eine Ausführungsform bevorzugt, bei welcher das Heizelement an der Innenseite der Fußbekleidung angeordnet, insbesondere angenäht oder angeklebt, ist. Vorzugsweise ist der elektrische Leiter zwischen Trägerlage und Fußbekleidung angeordnet, so dass der Leiter nicht direkt mit einem in der Fußbekleidung steckenden Fuß in Berührung kommt. Eine Ausführungsform, bei der das Heizelement an der Außenseite der Fußbekleidung angeordnet ist, ist ebenfalls denkbar.

Das Heizelement bzw. die Heizzone kann im Umlenkbereich breiter oder schmäler ausgeführt sein und/oder der elektrische Leiter kann in diesem Bereich auf oder in der Trägerlage dichter und/oder anders verlaufend, beispielsweise kreisförmig oder im Zickzackmuster, angeordnet sein.

In einer vorteilhaften Ausführungsform weist die Fußbekleidung eine Naht, insbesondere eine Kettelnaht, zum Verschließen der Fußbekleidung zwischen Umlenkbereich und Unterseite des Fußteiles auf. Andere Arten von Nähten, wie beispielsweise eine Rosso-Naht, oder andere Möglichkeiten die Fußbekleidung zu verschließen, wie beispielsweise Kleben, sind ebenso denkbar.

Vorzugsweise weist die Trägerlage des Heizelementes zumindest bereichsweise, insbesondere im Bereich der Anschlüsse und im Bereich des Überganges von der Zuleitung zur Heizzone, einer möglichen Längenveränderung der Trägerlage entgegenwirkende Aussteifungen auf. Dadurch sollen in diesen Bereichen angeordnete Verbindungen, wie beispielsweise Löt- oder Klemmverbindungen, vor mechanischer Beanspruchung, insbesondere vor Dehnung oder Stauchung, geschützt werden. Die Aussteifungen werden vorzugsweise dadurch gebildet, dass die Trägerlage in den entsprechenden Bereichen in einem vorerst flüssigen Mittel, wie beispielsweise Textilkleber, getränkt wird, welches im Anschluss daran aushärtet und somit die Trägerlage in besagten Bereichen versteift. Im Rahmen der Erfindung wäre es auch denkbar, diese Bereiche durch andere Vorkehrungen, wie beispielsweise besondere Nähte oder zusätzlich angeordnete Textillagen, vor mechanischer Beanspruchung, insbesondere vor Dehnung, zu schützen.

In einer bevorzugten Ausführungsform ist das Heizelement vom Umlenkbereich über die Oberseite des Fußteiles und anschließend am Schaft entlang, insbesondere seitlich entlang des Schaftes, bis zu einem dem Fußteil abgewandten Schaftende angeordnet. Ausführungsformen, bei denen das Heizelement vorne oder hinten am Schaft angeordnet ist und/oder sich das Heizelement nicht über die gesamte Länge des Schaftes oder nicht über den Fußteil hinaus oder nur entlang des Fußteiles erstreckt, sind ebenso denkbar.

Im Rahmen der Erfindung kann vorgesehen sein, die Anschlüsse für die Spannungsquelle im Bereich des Schaftendes, insbesondere in einem Umschlagbereich, anzuordnen. Dadurch kann die Fußbekleidung bei angeschlossener Spannungsquelle mit dem Umschlagbereich über die Spannungsquelle, die beispielsweise eine oder mehrere zusammengeschlossene Batterien aufweist, gestülpt werden, um so die Spannungsquelle zu schützen, zu verdecken oder in diesem Bereich zu fixieren.

In einer weiteren Ausführungsform ist die Fußbekleidung wenigstens bereichsweise, insbesondere am Schaft und/oder an der Unterseite des Fußteiles, verstärkt ausgeführt, beispielsweise indem bereichsweise ein oder mehrere zusätzliche Textillagen an dem Fußteil angeordnet sind oder indem die Fußbekleidung bereichsweise anders oder mit veränderten Einstellungen gefertigt ist.

Erfindungsgemäß ist weiters ein Verfahren zur Herstellung einer erfindungsgemäßen Fußbekleidung, insbesondere eines Sockens, vorgesehen, bei dem die Heizzone des Heizelements innen am Umlenkbereich angeordnet wird, so dass Zehen eines in der Fußbekleidung aufgenommenen Fußes vom Heizelement umschlossen werden.
Bevorzugt ist es, wenn die Fußbekleidung vom Schaftende bis zum Umlenkbereich in Form eines Schlauches gefertigt, insbesondere gestrickt, wird, so dass zwischen Unterseite des Fußteiles und Umlenkbereich eine Öffnung bleibt. Denkbar wäre jedoch auch, dass die Öffnung zwischen Oberseite des Fußteiles und Umlenkbereich oder am Ende des Umlenkbereichs bestehen bleibt und/oder dass die Fußbekleidung vom Umlenkbereich bis zum Schaftende gefertigt wird und/oder dass die Fußbekleidung nicht in Form eines Schlauches, sondern auf eine andere Art und/oder nicht in einem Stück, sondern aus mehreren Einzelteilen gefertigt wird.

Vorzugsweise wird im Anschluss daran das Heizelement an der Innenseite oder an der Außenseite der Fußbekleidung, insbesondere entlang der Oberseite des Fußteiles, angeordnet, insbesondere angenäht oder angeklebt, wofür die Fußbekleidung umgestülpt werden kann.

In einem weiteren bevorzugten Verfahrensschritt wird das Heizelement, insbesondere die Heizzone, im Umlenkbereich angeordnet, insbesondere angenäht oder angeklebt. Bevorzugt ist dabei, wenn im Wesentlichen die gesamte Innenfläche des Umlenkbereiches von dem Heizelement bedeckt ist. Es ist jedoch genauso denkbar, dass das Heizelement die Innenfläche des Umlenkbereiches nur bereichsweise abdeckt, vorzugsweise die Bereiche, die über und unter den Zehen eines Fußes angeordnet sind.

Vorzugsweise wird das Heizelement vom Umlenkbereich über die Oberseite des Fußteiles den Schaft entlang, insbesondere seitlich entlang, bis zum Schaftende angeordnet, insbesondere angenäht oder angeklebt. Denkbar ist jedoch auch eine Anordnung des Heizelementes in umgekehrter Reihenfolge.

Vorzugsweise wird die Öffnung, die nach der Fertigung der Fußbekleidung in Schlauchform bestehen bleibt, insbesondere zwischen der Unterseite des Fußteiles und dem Umlenkbereich, durch eine Naht, insbesondere eine Kettelnaht, verschlossen. Zum Verschließen dieser Öffnung können jedoch auch andere Nähte oder Methoden herangezogen werden.

Das Heizelement kann bereichsweise mit Aussteifungen oder anderen Verstärkungen gegen mechanische Belastungen versehen werden und/oder die Fußbekleidung kann durch Aufbringen zusätzlicher Textillagen oder durch Einsatz anderer oder veränderter Fertigungsmethoden zumindest bereichsweise verstärkt werden.

Ebenso können, vorzugsweise im Umschlagbereich, Anschlüsse für den elektrischen Leiter an oder in die Fußbekleidung angeordnet werden, beispielsweise als Druckknopfanschlüsse oder als Klemm- oder Steckverbindungen.

Weitere Einzelheiten, Merkmale und Vorteile der Erfindung ergeben sich aus der nachstehenden Beschreibung unter Bezugnahme auf die angeschlossenen Zeichnungen, in welcher bevorzugte Ausführungsformen der Erfindung dargestellt sind. Es zeigt:
- Fig. 1: eine herkömmliche Fußbekleidung in Seitenansicht,
- Fig. 2: eine erfindungsgemäße Fußbekleidung in Seitenansicht, und
- Fig. 3: eine erfindungsgemäße Fußbekleidung in Schrägansicht mit Freimachung im Bereich eines Fußteiles.

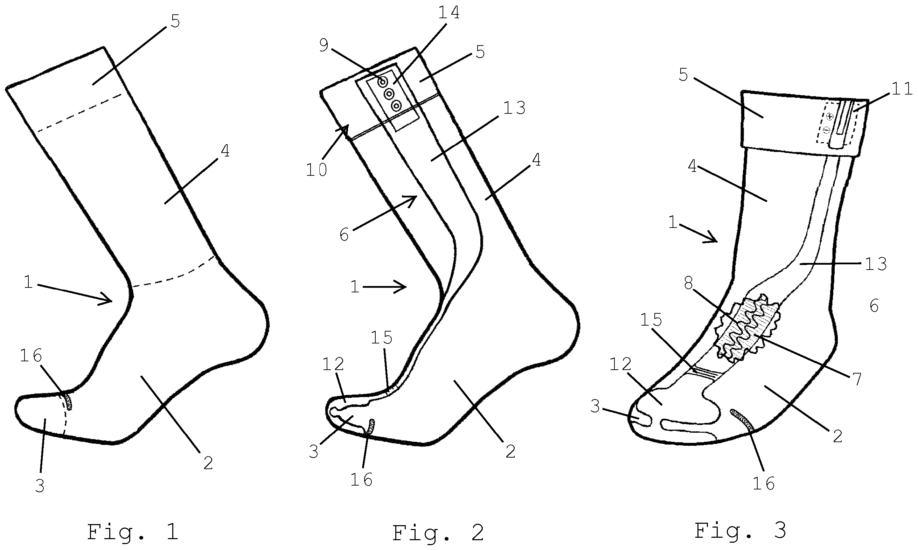

Fig. 1 zeigt eine herkömmliche Fußbekleidung 1, insbesondere einen Socken oder einen Strumpf. Die Fußbekleidung 1 weist einen Fußteil 2 auf, wobei an einem Ende des Fußteiles 2 ein Umlenkbereich 3 und an einem anderen Ende des Fußteiles 2 ein Schaft 4 mit Schaftende 5 angeordnet ist. Eine Naht, insbesondere eine Kettelnaht 16 verschließt eine beim Fertigen der Fußbekleidung 1 entstehende Öffnung zwischen einer Oberseite des Fußteiles 2 und dem Umlenkbereich 3. Weist die herkömmliche Fußbekleidung 1 ein Heizelement 6 auf, so ist dieses an einer Unterseite des Fußteiles 2 angeordnet. Die Unterseite des Fußteiles 2 ist in Gebrauchslage der Fußbekleidung 1 an der Fußsohle angeordnet.

Fig. 2 und 3 zeigen eine erfindungsgemäße Fußbekleidung 1, die wie die herkömmliche Fußbekleidung 1 einen Fußteil 2, einen Umlenkbereich 3, einen Schaft 4 und ein Schaftende 5 mit Umschlagbereich 10 umfasst.

Ein Heizelement 6 ist innen in der Fußbekleidung 1 angeordnet, und weist eine Trägerlage 7 auf, in die ein elektrischer Leiter 8 wellenförmig eingewirkt ist. Das Heizelement 6 verläuft vom Schaftende 5 seitlich entlang des Schafts 4 über die Oberseite des Fußteiles 2 bis zum Umlenkbereich 3, wobei der elektrische Leiter 8 zwischen Trägerlage 7 des Heizelementes 6 und Fußbekleidung 1 angeordnet ist. Am Schaftende 5 im Umschlagbereich 10 weist das Heizelement 6 Anschlüsse 9 für eine Spannungsquelle 11 auf, die bei nicht umgeschlagenem Umschlagbereich 5 außen an der Fußbekleidung 1 am Schaftende 5 angeordnet sind.

Der elektrische Leiter 8 bildet eine Zuleitung 13 zu einer Heizzone 12 des Heizelementes 6, wobei in der Heizzone 12 wenigstens ein Heizdraht angeordnet ist. Das Heizelement 6 umfasst weiters einen Bereich 14, in dem die Anschlüsse 9 angeordnet sind, und einen Übergang 15 zwischen der Zuleitung 13 und der Heizzone 12, in dem eine Verbindung zwischen elektrischem Leiter 8 und Heizdraht angeordnet ist. Die Heizzone 12 des Heizelementes 6 ist innen am Umlenkbereich 3 angeordnet, so dass Zehen eines in der Fußbekleidung 1 aufgenommenen Fußes vom Heizelement 6 umschlossen werden.

Der Bereich 14 der Anschlüsse 9 und der Übergang 15 von der Zuleitung 13 zur Heizzone 12 sind versteift ausgeführt, um darin angeordnete Verbindungen zwischen elektrischem Leiter 8 und Anschlüssen 9 und elektrischem Leiter 8 der Heizzone 12 und elektrischem Leiter 8 der Zuleitung 13 vor mechanischen Beanspruchungen, wie beispielsweise vor Dehnung, zu schützen.

Fig. 3 zeigt die Fußbekleidung 1 mit umgeschlagenem Schaftende 5, wobei die an die Anschlüsse 9 angeschlossene Spannungsquelle 11 zwischen dem nach außen umgeschlagenen Schaftende 5 und dem Schaft 4 angeordnet ist.

Die erfindungsgemäße Fußbekleidung 1 ist, anders als die herkömmliche Fußbekleidung 1, zwischen Unterseite des Fußteiles 2 und Umlenkbereich 3 und nicht zwischen Oberseite des Fußteiles 2 und Umlenkbereich 3 durch eine Kettelnaht 16 verschlossen.

Zusammenfassend kann ein Ausführungsbeispiel der Erfindung wie folgt dargestellt werden:
Eine Fußbekleidung 1, insbesondere ein Socken, umfasst einen Schaft 4 mit einem Schaftende 5 zur Aufnahme eines Unterbeines, einen am Schaft 4 angeordneten Fußteil 2 mit einer Oberseite und einer Unterseite zur Aufnahme eines Fußes, und einen am Fußteil 2 angeordneten Umlenkbereich 3 zur Aufnahme von Zehen. Die Fußbekleidung 1 weist wenigstens ein Heizelement 6 mit Anschlüssen 9 für eine Spannungsquelle 11 auf, wobei das Heizelement 6 im Bereich des Fußteiles 2 an der Oberseite des Fußteiles 2 angeordnet ist. Das Heizelement 6 verläuft von der Oberseite des Fußteiles 2 in den Umlenkbereich 3 zur Aufnahme der Zehen und ist im Umlenkbereich im vorderen Zehenspitzenbereich zur Unterseite des Fußteils 2 umgelenkt.

## Patentansprüche

1. Fußbekleidung (1), insbesondere Socken, umfassend einen Schaft (4) mit einem Schaftende (5) zur Aufnahme eines Unterbeines, einen am Schaft (4) angeordneten Fußteil (2) mit einer Oberseite und einer Unterseite zur Aufnahme eines Fußes, und einen am Fußteil (2) angeordneten Umlenkbereich (3) zur Aufnahme von Zehen, wobei die Fußbekleidung (1) wenigstens ein Heizelement (6) mit Anschlüssen (9) für eine Spannungsquelle (11) aufweist, und wobei das Heizelement (6) im Bereich des Fußteiles (2) an der Oberseite des Fußteiles (2) angeordnet ist und eine Heizzone (12) aufweist, **dadurch gekennzeichnet, dass** die Heizzone (12) des Heizelements (6) innen am Umlenkbereich (3) angeordnet ist, so dass Zehen eines in der Fußbekleidung (1) aufgenommenen Fußes vom Heizelement (6) umschlossen werden.

2. Fußbekleidung (1) nach Anspruch 1, **dadurch gekennzeichnet, dass** das Heizelement (6) wenigstens eine, vorzugsweise elastische und in eine Richtung längenveränderbare, Trägerlage (7) aufweist, und dass wenigstens ein elektrischer Leiter (8), vorzugsweise zumindest bereichsweise wellenförmig, an der Trägerlage (7) angeordnet ist, wobei insbesondere der elektrische Leiter (8) zwischen der Trägerlage (7) und der Fußbekleidung (1) angeordnet ist.

3. Fußbekleidung (1) nach Anspruch 2, **dadurch gekennzeichnet, dass** der elektrische Leiter (8) des Heizelementes (6) mit der Trägerlage (7) verbunden ist, insbesondere auf der Trägerlage (7) aufgestickt und/oder aufgeklebt und/oder an einer Oberfläche und/oder in die Trägerlage (7) eingewebt und/oder eingewirkt ist.

4. Fußbekleidung (1) nach Anspruch 2 oder 3, **dadurch gekennzeichnet, dass** das Heizelement (6) aus einer Zuleitung (13) und einer Heizzone (12) besteht, wobei der elektrische Leiter (8) des Heizelementes (6) im Bereich der Zuleitung (13) einen vorzugsweise niedrigeren ohmschen Widerstand aufweist als im Bereich der Heizzone (12).

5. Fußbekleidung (1) nach einem der Ansprüche 1 bis 4, **dadurch gekennzeichnet, dass** das Heizelement (6) an der Innenseite oder an der Außenseite der Fußbekleidung (1) angeordnet, insbesondere angenäht oder angeklebt, ist.

6. Fußbekleidung (1) nach Anspruch 4 oder 5, **dadurch gekennzeichnet, dass** die Heizzone (12) im Umlenkbereich (3) im Zehenspitzenbereich angeordnet ist, wobei das Heizelement (6) bzw. die Heizzone (12) im Umlenkbereich (3) breiter oder schmäler ausgeführt ist.

7. Fußbekleidung (1) nach einem der Ansprüche 1 bis 6, **dadurch gekennzeichnet, dass** eine Naht, insbesondere eine Kettelnaht (16), zum Verschließen der Fußbekleidung (1) zwischen Umlenkbereich (3) und Unterseite des Fußteiles (2) angeordnet ist.

8. Fußbekleidung (1) nach einem der Ansprüche 4 bis 7, **dadurch gekennzeichnet, dass** die Trägerlage (7) des Heizelementes (6) zumindest bereichsweise, insbesondere in einem Bereich (14) der Anschlüsse (9) und in einem weiteren Bereich (15) des Überganges von der Zuleitung (13) zur Heizzone (12), der Längenveränderung der Trägerlage (7) entgegenwirkende Aussteifungen aufweist.

9. Fußbekleidung (1) nach einem der Ansprüche 1 bis 8, **dadurch gekennzeichnet, dass** das Heizelement (6) vom Umlenkbereich (3) über die Oberseite des Fußteiles (2) am Schaft (4) entlang, insbesondere seitlich entlang, bis zum Schaftende (5) angeordnet ist.

10. Fußbekleidung (1) nach einem der Ansprüche 1 bis 9, **dadurch gekennzeichnet, dass** die Anschlüsse (9) für die Spannungsquelle (11) im Bereich (14) des Schaftendes (5), insbesondere in einem Umschlagbereich (10), angeordnet sind.

11. Verfahren zur Herstellung einer Fußbekleidung (1), nach einem der Ansprüche 1 bis 10, insbesondere eines Sockens, **dadurch gekennzeichnet, dass** die Heizzone (12) des Heizelements (6) innen am Umlenkbereich (3) angeordnet wird so dass Zehen eines in der Fußbekleidung (1) aufgenommenen Fußes vom Heizelement (6) umschlossen werden.

12. Verfahren nach Anspruch 11, **dadurch gekennzeichnet, dass** die Fußbekleidung (1) vom Schaftende (5) bis zum Umlenkbereich (3) in Form eines Schlauches gefertigt, insbesondere gestrickt, wird, so dass zwischen Unterseite des Fußteiles (2) und Umlenkbereich (3) eine Öffnung bleibt.

13. Verfahren nach Anspruch 11 oder 12, **dadurch gekennzeichnet, dass** die Heizzone (12) im Umlenkbereich (3) im Zehenspitzenbereich umgelenkt wird.

14. Verfahren nach einem der Ansprüche 11 bis 13, **dadurch gekennzeichnet, dass** das Heizelement (6), insbesondere die Heizzone (12), im Umlenkbereich (3) angeordnet, insbesondere angenäht oder angeklebt, wird, insbesondere dass das Heizelement (6) vom Umlenkbereich (3) über die Oberseite des Fußteiles (2) den Schaft (4) entlang, insbesondere seitlich entlang, bis zum Schaftende (5) angeordnet, insbesondere angenäht oder angeklebt, wird.

15. Verfahren nach einem der Ansprüche 12 bis 14, **dadurch gekennzeichnet, dass** die Öffnung zwischen der Unterseite des Fußteiles (2) und dem Umlenkbereich (3) durch eine Naht, insbesondere eine Kettelnaht (16), verschlossen wird.

## Claims

1. Footwear (1), in particular a sock, comprising a shank (4) having a shank end (5) for accommodating a lower leg, a foot part (2) arranged on the shank (4) with a top and a bottom for accommodating a foot, and a deflection region (3) arranged on the foot part (2) for accommodating toes, wherein the footwear (1) has at least one heating element (6) with connections (9) for a voltage source (11), and wherein the heating element (6) in the region of the foot part (2) is arranged on the top of the foot part (2) and has a heating zone (12), **characterized in that** the heating zone (12) of the heating element (6) is arranged internally at the deflection region (3) such that toes of a foot accommodated in the footwear (1) are surrounded by the heating element (6).

2. Footwear (1) according to claim 1, **characterized in that** the heating element (6) has at least one carrier layer (7) which is preferably elastic and the length of which can be changed in one direction, and **in that** at least one electrical conductor (8) is arranged, preferably at least in some regions in an undulatory manner, on the carrier layer (7), wherein in particular the electrical conductor (8) is arranged between the carrier layer (7) and the footwear (1).

3. Footwear (1) according to claim 2, **characterized in that** the electrical conductor (8) of the heating element (6) is connected to the carrier layer (7), in particular is stitched and/or glued onto the carrier layer (7) and/or is woven and/or knitted onto a surface and/or into the carrier layer (7).

4. Footwear (1) according to claim 2 or 3, **characterized in that** the heating element (6) consists of a supply line (13) and a heating zone (12), wherein the electrical conductor (8) of the heating element (6) has a preferably lower ohmic resistance in the region of the supply line (13) than in the region of the heating zone (12).

5. Footwear (1) according to any one of claims 1 to 4, **characterized in that** the heating element (6) is arranged on the inner side or on the outer side of the footwear (1), in particular by sewing or gluing.

6. Footwear (1) according to claim 4 or 5, **characterized in that** the heating zone (12) in the deflection region (3) is arranged in the region of the tips of the toes, wherein the heating element (6) or the heating zone (12) is designed to be broader or narrower in the deflection region (3).

7. Footwear (1) according to any one of claims 1 to 6, **characterized in that** a seam, in particular a whipped seam (16), for closing the footwear (1) is arranged between the deflection region (3) and the lower side of the foot part (2) .

8. Footwear (1) according to any one of claims 4 to 7, **characterized in that**, at least in some regions, in particular in a region (14) of the connections (9) and in a further region (15) of the transition from the supply line (13) to the heating zone (12), the carrier layer (7) of the heating element (6) has stiffeners which prevent the change in length of the carrier layer (7).

9. Footwear (1) according to any one of claims 1 to 8, **characterized in that** the heating element (6) is arranged from the deflection region (3), over the top of the foot part (2), along the shank (4), in particular along the side of the latter, to the shank end (5).

10. Footwear (1) according to any one of claims 1 to 9, **characterized in that** the connections (9) for the voltage source (11) are arranged in the region (14) of the shank end (5), in particular in a fold-over region (10).

11. Method for producing a footwear (1) according to any one of claims 1 to 10, in particular a sock, **characterized in that** the heating zone (12) of the heating element (6) is arranged internally at the deflection region (3) such that toes of a foot accommodated in the item of footwear (1) are surrounded by the heating element (6).

12. Method according to claim 11, **characterized in that** the footwear (1) from the shank end (5) to the deflection region (3) is manufactured, in particular knitted, in the form of a tube such that an opening remains between the lower side of the foot part (2) and the deflection region (3) .

13. Method according to claim 11 or 12, **characterized in that** the heating zone (12) in the deflection region (3) is deflected in the region of the tips of the toes.

14. Method according to any one of claims 11 to 13, **characterized in that** the heating element (6), in particular the heating zone (12), is arranged in the deflection region (3), in particular by sewing or gluing, in particular **in that** the heating element (6) is arranged, in particular by sewing or gluing, from the deflection region (3), over the top of the foot part (2), along the shank (4), in particular along the side of the latter, to the shank end (5).

15. Method according to any one of claims 12 to 14, **characterized in that** the opening between the bottom side of the foot part (2) and the deflection region (3) is closed by a seam, in particular a whipped seam (16).

## Revendications

1. Article chaussant (1), en particulier chaussettes, comprenant une tige (4) avec une extrémité de tige (5) destinée à recevoir un bas de jambe, une partie pied (2) disposée sur la tige (4), qui présente un côté supérieur et un côté inférieur et est destinée à recevoir un pied, et une zone de déviation (3) disposée sur la partie pied (2) et destinée à recevoir des orteils, dans lequel l'article chaussant (1) présente au moins un élément chauffant (6) avec des connexions (9) pour une source de tension (11), et dans lequel l'élément chauffant (6) est disposé dans la zone de la partie pied (2) sur le côté supérieur de la partie pied (2) et présente une zone chauffante (12), **caractérisé en ce que** la zone chauffante (12) de l'élément chauffant (6) est disposée du côté intérieur dans la zone de déviation (3), de telle sorte que les orteils d'un pied reçu dans l'article chaussant (1) sont entourés par l'élément chauffant (6).

2. Article chaussant (1) selon la revendication 1, **caractérisé en ce que** l'élément chauffant (6) présente au moins une couche de support (7), de préférence élastique, de longueur variable dans une direction, et qu'au moins un conducteur électrique (8) est disposé sur la couche de support (7), de préférence de manière ondulée au moins dans certaines zones, dans lequel le conducteur électrique (8) est en particulier disposé entre la couche de support (7) et l'article chaussant (1) .

3. Article chaussant (1) selon la revendication 2, **caractérisé en ce que** le conducteur électrique (8) de l'élément chauffant (6) est relié à la couche de support (7), en particulier est brodé et/ou collé sur la couche de support (7) et/ou tissé et/ou tricoté sur une surface et/ou dans la couche de support (7) .

4. Article chaussant (1) selon la revendication 2 ou 3, **caractérisé en ce que** l'élément chauffant (6) est constitué d'une ligne d'alimentation (13) et d'une zone chauffante (12), le conducteur électrique (8) de l'élément chauffant (6) ayant une résistance ohmique de préférence plus faible au niveau de la ligne d'alimentation (13) qu'au niveau de la zone chauffante (12).

5. Article chaussant (1) selon l'une des revendications 1 à 4, **caractérisé en ce que** l'élément chauffant (6) est disposé, en particulier cousu ou collé, du côté intérieur ou du côté extérieur de l'article chaussant (1).

6. Article chaussant (1) selon la revendication 4 ou 5, **caractérisé en ce que** la zone chauffante (12) est disposée dans la zone de déviation (3) au niveau de la pointe des pieds, l'élément chauffant (6) ou la zone chauffante (12) étant réalisé(e) plus large ou plus étroit(e) dans la zone de déviation (3).

7. Article chaussant (1) selon l'une des revendications 1 à 6, **caractérisé en ce qu'**une couture, en particulier une couture de remaillage (16), pour la fermeture de l'article chaussant (1) est disposée entre la zone de déviation (3) et le côté inférieur de la partie pied (2).

8. Article chaussant (1) selon l'une des revendications 4 à 7, **caractérisé en ce que** la couche de support (7) de l'élément chauffant (6) présente, au moins dans certaines zones, en particulier dans une zone (14) des connexions (9) et dans une autre zone (15) de la transition entre la ligne d'alimentation (13) et la zone chauffante (12), des renforcements qui s'opposent à la modification de longueur de la couche de support (7).

9. Article chaussant (1) selon l'une des revendications 1 à 8, **caractérisé en ce que** l'élément chauffant (6) est disposé de la zone de déviation (3) à l'extrémité de tige (5), en passant par le côté supérieur de la partie pied (2), le long de la tige (4), en particulier latéralement le long de la tige (4).

10. Article chaussant (1) selon l'une des revendications 1 à 9, **caractérisé en ce que** les connexions (9) pour la source de tension (11) sont disposées dans la zone (14) de l'extrémité de tige (5), en particulier dans une zone de retournement (10) .

11. Procédé de fabrication d'un article chaussant (1) selon l'une des revendications 1 à 10, en particulier d'une chaussette, **caractérisé en ce que** la zone chauffante (12) de l'élément chauffant (6) est disposée du côté intérieur dans la zone de déviation (3), de telle sorte que les orteils d'un pied reçu dans l'article chaussant (1) sont entourés par l'élément chauffant (6).

12. Procédé selon la revendication 11, **caractérisé en ce que** l'article chaussant (1) est fabriqué, en particulier tricoté, sous la forme d'un tube allant de l'extrémité de tige (5) à la zone de déviation (3), de sorte qu'il reste une ouverture entre le côté inférieur de la partie pied (2) et la zone de déviation (3).

13. Procédé selon la revendication 11 ou 12, **caractérisé en ce que** la zone chauffante (12) est déviée dans la zone de déviation (3) au niveau de la pointe des pieds.

14. Procédé selon l'une des revendications 11 à 13, **caractérisé en ce que** l'élément chauffant (6), en particulier la zone chauffante (12), est disposé, en particulier cousu ou collé, dans la zone de déviation (3), en particulier que l'élément chauffant (6) est disposé, en particulier cousu ou collé, de la zone de déviation (3) à l'extrémité de tige (5), en passant par le côté supérieur de la partie pied (2), le long de la tige (4), en particulier latéralement le long de la tige (4).

15. Procédé selon l'une des revendications 12 à 14, **caractérisé en ce que** l'ouverture entre le côté inférieur de la partie pied (2) et la zone de déviation (3) est fermée par une couture, en particulier une couture de remaillage (16).
